# EUROPÄISCHE PATENTSCHRIFT

(11) **EP 3 398 515 B1**
(45) Veröffentlichungstag und Bekanntmachung des Hinweises auf die Patenterteilung: **24.06.2020**
(21) Anmeldenummer: 17169269.2
(22) Anmeldetag: 03.05.2017
(51) Int. Cl.: A61B 6/00, G06T 11/00, A61B 6/03

(54) **ADAPTIVES VERFAHREN ZUM ERZEUGEN VON ARTEFAKTREDUZIERTEN CT-BILDDATEN, SOWIE BILDDATENREKONSTRUKTIONSEINHEIT UND ENTSPRECHENDES COMPUTERPROGRAMMPRODUKT.**
ADAPTIVE METHOD FOR GENERATING OF CT IMAGE DATA WITH REDUCED ARTEFACTS, AS WELL AS IMAGE RECONSTRUCTION UNIT AND CORRESPONDING COMPUTER PROGRAM PRODUCT.
PROCÉDÉ ADAPTATIF DE PRODUCTION DE DONNÉES D'IMAGE CT À ARTÉFACT RÉDUIT ET UNITÉ DE RECONSTRUCTION D'IMAGE ET PRODUIT DE PROGRAMME INFORMATIQUE ASSOCIÉ

(43) Veröffentlichungstag der Anmeldung: 07.11.2018
(73) Patentinhaber: Siemens Healthcare GmbH, 91052 Erlangen (DE)
(72) Erfinder: Feuerlein, Ute, 91052 Erlangen (DE); Hofmann, Christian, 91052 Erlangen (DE); Mayer, Robert, 91207 Lauf/Schönberg (DE); Raupach, Rainer, 91336 Heroldsbach (DE); Soza, Grzegorz, 90562 Heroldsberg (DE)

(56) Entgegenhaltungen:
- EP-A1- 2 998 729
- EP-A1- 3 219 260
- DE-A1-102015 014 908

## Beschreibung

Die Erfindung betrifft ein adaptives Verfahren zum Erzeugen von CT-Bilddaten. Überdies betrifft die Erfindung eine Bilddatenrekonstruktionseinrichtung. Ferner betrifft die Erfindung ein Computertomographiesystem.

Ein in der Medizin häufig eingesetztes Bildgebungsverfahren ist die Computertomographie (kurz CT). Die Computertomographie basiert auf der Erfassung von Röntgenstrahlung, wobei sogenannte Projektionsmessdaten erzeugt werden. Bei CT-Systemen läuft gewöhnlich eine an einer Gantry angeordnete Kombination aus Röntgenquelle und gegenüberliegend angeordnetem Röntgendetektor um einen Messraum um, in dem sich das Untersuchungsobjekt (das im Folgenden ohne Beschränkung der Allgemeinheit als Patient bezeichnet wird) befindet. Das Drehzentrum (auch "Isozentrum" genannt) fällt dabei mit einer sogenannten Systemachse z zusammen. Bei einem oder mehreren Umläufen wird der Patient mit Röntgenstrahlung der Röntgenquelle durchstrahlt, wobei mit Hilfe des gegenüberliegenden Röntgendetektors Projektionsmessdaten bzw. Röntgenprojektionsdaten erfasst werden. Auf Basis der Projektionsmessdaten werden dann Bilddaten rekonstruiert, welche dem Benutzer zur Begutachtung angezeigt werden.

Die erzeugten Projektionsmessdaten sind insbesondere von der Bauart des Röntgendetektors abhängig. Röntgendetektoren weisen gewöhnlich eine Mehrzahl von Detektionseinheiten auf, die meist in Form eines regelmäßigen Pixelarrays angeordnet sind. Die Detektionseinheiten erzeugen jeweils für auf die Detektionseinheiten auftreffende Röntgenstrahlung ein Detektionssignal, welches zu bestimmten Zeitpunkten hinsichtlich Intensität und spektraler Verteilung der Röntgenstrahlung analysiert wird, um Rückschlüsse auf das Untersuchungsobjekt zu erhalten und Projektionsmessdaten zu erzeugen.

Bei der Rekonstruktion von Bilddaten auf Basis der Projektionsmessdaten treten häufig Artefakte, insbesondere Metallartefakte auf. Diese Störeffekte werden in der Regel durch Implantate bzw. Prothesen im Körper des untersuchten Patienten verursacht. Beispielsweise können Zahnimplantate bei einer Schädelaufnahme zu Artefakten führen, auch wenn sie selbst außerhalb des eigentlichen Aufnahmebereichs, beispielsweise des Bereichs des Gehirns, liegen.

Zur Reduktion von Artefakten in der Bilddarstellung können spezielle Rekonstruktionen durchgeführt werden. Oft wird erst nach einer Bildaufnahme und einer Sichtung der dabei rekonstruierten Bilddaten festgestellt, dass eine artefaktreduzierte Rekonstruktion benötigt wird. Dazu müssen die erzeugten Bilddatensätze erst gesichtet werden und dann entschieden werden, ob eine zusätzliche artefaktreduzierte Rekonstruktion sinnvoll ist oder nicht. Der Benutzer muss dann eine solche zusätzliche artefaktreduzierte Rekonstruktion anfordern. Infolge der zusätzlichen Rekonstruktion werden das gesamte Datenvolumen und der benötigte Zeitaufwand stark erhöht.

In DE 10 2015 014 908 A1 wird eine medizinische Bildverarbeitungsvorrichtung beschrieben. Die medizinische Bildverarbeitungsvorrichtung weist eine erste Akquirierungseinheit, eine zweite Akquirierungseinheit und eine Erzeugungseinheit eines Bilds mit entferntem Teil auf. Dabei ist die erste Akquirierungseinheit angepasst, um eine erste Röntgenaufnahme zu akquirieren, die eine virtuelle Röntgenaufnahme darstellt, die erzeugt ist, um einen spezifizierten Teil oder einen vorbestimmten Teil unter Teilen, die in Volumendatendaten enthalten sind, die eine dreidimensionale Struktur des Inneren eines Körpers eines Patienten anzeigen, hervorzuheben. Die zweite Akquirierungseinheit ist angepasst, um eine zweite Röntgenaufnahme des Inneren des Körpers des Patienten zu akquirieren, und die Erzeugungseinheit eines Bildes mit entferntem Teil ist angepasst, um ein Bild mit entferntem Teil zu erzeugen, indem der spezifizierte oder vorbestimmte Teil oder andere Teile als der spezifizierte oder vorbestimmte Teil aus der zweiten Röntgenaufnahme mit Bezug auf die erste Röntgenaufnahme entfernt wird bzw. werden.

Es besteht also das Problem, qualitativ gute Bilddaten mit einem möglichst geringen zeitlichen und rechnerischen Aufwand zu erzeugen.

Diese Aufgabe wird durch ein adaptives Verfahren zum Erzeugen von CT-Bilddaten gemäß Patentanspruch 1, eine Bilddatenrekonstruktionseinrichtung gemäß Patentanspruch 11 und ein Computertomographiesystem gemäß Patentanspruch 12 gelöst.

Bei dem erfindungsgemäßen adaptiven Verfahren zum Erzeugen von CT-Bilddaten werden Projektionsmessdaten von einem Untersuchungsbereich eines Untersuchungsobjekts akquiriert. Weiterhin werden unkorrigierte Bilddaten von dem Untersuchungsbereich erzeugt. Der Untersuchungsbereich kann zum Beispiel ein Körperbereich eines Patienten sein. Der Untersuchungsbereich kann aber für den Fall einer nichtmedizinischen Anwendung auch Teil eines unbelebten Objekts sein. Die unkorrigierten Bilddaten können zum Beispiel auf Basis der Projektionsmessdaten rekonstruiert werden, sie können aber auch Bilddaten umfassen, welche durch Aufnehmen eines Topogramms oder durch das Erzeugen von RTD-Daten (RTD = Real Time Display Data), d.h. Echtzeitbilddaten, manchmal auch als Rohbilddaten bezeichnet, gewonnen werden. Allgemein sollen als "unkorrigierte" Bilddaten Bilddaten von dem Untersuchungsbereich verstanden werden, welche noch keiner Artefaktkorrektur unterzogen wurden und Bildartefakte, beispielsweise Metallartefakte, aufweisen können.

Dann werden artefaktbehaftete Teilbereiche des Untersuchungsbereichs auf Basis zumindest eines Teils der unkorrigierten Bilddaten ermittelt. D.h., es wird insbesondere ermittelt, ob und gegebenenfalls in welchem Teilbereich des Untersuchungsbereichs Artefakte in den unkorrigierten Bilddaten auftreten. Anders ausgedrückt werden die Positionen der Artefakte und deren Ausdehnung im Untersuchungsbereich ermittelt und Teilbereiche festgelegt, die die Artefakte enthalten. Eine solche Ermittlung von Artefakten kann anhand charakteristischer Eigenschaften der ermittelten Bilddaten und Projektionsmessdaten, wie zum Beispiel einer Abschwächung bzw. eines Dichtewertes in einem bestimmten Bereich erfolgen.

Weiterhin wird eine artefaktreduzierte Bildrekonstruktion in den artefaktbehafteten Teilbereichen des Untersuchungsbereichs durchgeführt, wobei artefaktreduzierte Teilbilddaten nur von den artefaktbehafteten Teilbereichen erzeugt werden. Artefakte können für den Fall, dass es sich bei den Artefakten um Metallartefakte handelt, zum Beispiel mit Hilfe eines sogenannten iMAR-Rekonstruktionsverfahrens korrigiert werden (iMAR = iterative Metal Artifact Reduction). Allgemein soll eine artefaktreduzierte Bildrekonstruktion eine Bildrekonstruktionsverfahren umfassen, mit dem Bildartefakte reduziert werden können.

Schließlich werden artefaktreduzierte Bilddaten von dem gesamten Untersuchungsbereich durch Kombination zumindest eines Teils der unkorrigierten Bilddaten und der artfaktreduzierten Teilbilddaten erzeugt. Die für die Kombination verwendeten unkorrigierten Bilddaten können zum Beispiel unkorrigierte Bilddaten sein, welche bei dem Schritt des Erzeugens von unkorrigierten Bilddaten von dem Untersuchungsbereich auf Basis der akquirierten Projektionsmessdaten rekonstruiert wurden. Die Ermittlung der Bereiche bzw. Teilbereiche mit Artefakten erfolgt vorzugsweise nicht nach, sondern schon während der Rekonstruktion von unkorrigierten Bilddaten.

Für den Fall, dass bei dem Schritt des Erzeugens von unkorrigierten Bilddaten auch "provisorische" Bilddaten, wie zum Beispiel Topogramme oder RTD-Bilddaten, erzeugt wurden, d.h. die Festlegung der Teilbereiche mit zu korrigierenden Artefakten auf Basis dieser "provisorischen" Bilddaten erfolgte, werden bei dem erfindungsgemäßen Verfahren noch zusätzlich zu den "provisorischen" Bilddaten auf Basis der akquirierten Projektionsmessdaten unkorrigierte Bilddaten rekonstruiert, wobei zumindest ein Teil dieser rekonstruierten unkorrigierten Bilddaten dann mit den artefaktreduzierten Teilbilddaten zu artefaktreduzierten Bilddaten kombiniert werden. Insbesondere werden die unkorrigierten Bilddaten, welche auf Basis der akquirierten Projektionsmessdaten rekonstruiert wurden, als Bilddaten in den Teilbereichen des Untersuchungsbereichs verwendet, in denen keine Artefakte aufgefunden wurden.

Prinzipiell ist das erfindungsgemäße Verfahren unabhängig vom eingesetzten Korrekturalgorithmus einsetzbar. Notwendig ist einer Lokalisierung von Artefakten bzw. von Regionen, auf die eine Artefaktbildung beschränkt ist.

Vorteilhaft kann für einige Korrekturverfahren das Rekonstruieren dieser unkorrigierten Bilddaten auf Bereiche, in denen keine Artefakte aufgefunden wurden, beschränkt werden, wodurch der Bilderzeugungsprozess bzw. Bildrekonstruktionsprozess weiter beschleunigt werden kann.

Die Entscheidung, ob und in welchen Teilbereichen eine Artefaktkorrektur erfolgt, wird vorzugsweise automatisiert getroffen. Auf diese Weise erhält der Benutzer immer einen optimalen Datensatz, ohne spezielle Kenntnisse haben zu müssen, um Artefakte auffinden zu können. Durch eine intelligente Auswahl der Bildbereiche, die eine Artefaktkorrektur, insbesondere eine Metallartefaktkorrektur, benötigen, kann eine Verbesserung der Bildqualität erreicht werden und trotzdem die bei der Erzeugung von Bilddaten zu verarbeitende Datenmenge vorteilhaft reduziert werden, wodurch der Gesamtablauf des Bildgebungsprozesses beschleunigt wird. Das Verfahren kann auch auf andere Artefakte als Metallartefakte angewendet werden.

Ein Beispiel für ein Verfahren zur Korrektur von Bewegungsartefakten ist das iTRIM-Verfahren (intelligent time resolution improvement method = intelligentes Verfahren zur Verbesserung der zeitlichen Auflösung). Mit diesem Verfahren wird eine Verbesserung einer Zeitauflösung in Regionen, in denen man diese Bewegungsartefakte sieht, erreicht. Vorteilhaft kann ein solches Verfahren bei einer Thorax-Abbildung auf Herzregionen, in denen man die Bewegungsartefakte sieht, beschränkt werden.

Bei der erwähnten iMAR-Rekonstruktion ist es sinnvoll, das unkorrigierte Volumen sowieso immer als Referenz zu erzeugen, da der Algorithmus in seltenen Fällen auch einmal fehlerhaft korrigieren kann.

Werden aber andere Korrekturalgorithmen als iMAR in dem erfindungsgemäßen Verfahren eingesetzt, bei denen man die unkorrigierten Bilder nicht für die artefaktbehafteten Teilbereiche des Untersuchungsbereichs benötigt, so können diese eingespart werden, d.h. es kann auf eine Rekonstruktion von unkorrigierten Bilddaten in artefaktbehafteten Teilbereich verzichtet werden.

In diesem Fall reicht es, einen einzigen Datensatz bzw. Bilddatensatz auf Basis der beiden sich ergänzenden rekonstruierten Bilddaten zu erzeugen. Bei dieser Version bzw. diesen anderen Korrekturverfahren, wie zum Beispiel iTRIM, hat man die geringste Menge an Bilddaten und den geringsten Rekonstruktionsaufwand, da kein Bildbereich zweimal rekonstruiert wird.

Die erfindungsgemäße Bilddatenrekonstruktionseinrichtung weist eine Eingangsschnittstelle zum Akquirieren von Projektionsmessdaten von einem Untersuchungsbereich eines Untersuchungsobjekts auf. Teil der erfindungsgemäßen Bilddatenrekonstruktionseinrichtung ist auch eine Rekonstruktionseinheit zum Rekonstruieren von unkorrigierten Bilddaten auf Basis der Projektionsmessdaten. Die erfindungsgemäße Bilddatenrekonstruktionseinrichtung umfasst zudem eine Teilbereichsermittlungseinheit zum Ermitteln von artefaktbehafteten Teilbereichen des Untersuchungsbereichs auf Basis von unkorrigierten Bilddaten. Ferner weist die erfindungsgemäße Bilddatenrekonstruktionseinrichtung eine Korrektureinheit zum Durchführen einer artefaktreduzierten Bildrekonstruktion in den artefaktbehafteten Teilbereichen des Untersuchungsbereichs auf, wobei artfaktreduzierte Teilbilddaten nur von den artefaktbehafteten Teilbereichen erzeugt werden. Teil der erfindungsgemäßen Bilddatenrekonstruktionseinrichtung ist auch eine Bilderzeugungseinheit zum Erzeugen von artefaktreduzierten Bilddaten von dem gesamten Untersuchungsbereich durch Kombination zumindest eines Teils der rekonstruierten unkorrigierten Bilddaten und der artfaktreduzierten Teilbilddaten. Die erzeugten artefaktreduzierten Bilddaten werden dann an eine Bildanzeigeeinrichtung oder eine Bildspeichereinheit weitergegeben, wo sie entweder angezeigt oder für eine weitere Verarbeitung oder Übermittlung an andere Einheiten gespeichert werden.

Das erfindungsgemäße Computertomographiesystem weist eine Scaneinheit zum Erfassen eines zu untersuchenden Bereichs eines Untersuchungsobjekts, eine Steuereinrichtung zum Ansteuern der Scaneinheit und die erfindungsgemäße Bilddatenrekonstruktionseinrichtung auf.

Die wesentlichen Komponenten der erfindungsgemäßen Bilddatenrekonstruktionseinrichtung können zum überwiegenden Teil in Form von Softwarekomponenten ausgebildet sein. Dies betrifft insbesondere Teile der Rekonstruktionseinheit, der Teilbereichsermittlungseinheit, der Korrektureinheit und der Bilderzeugungseinheit. Grundsätzlich können diese Komponenten aber auch zum Teil, insbesondere wenn es um besonders schnelle Berechnungen geht, in Form von softwareunterstützter Hardware, beispielsweise FPGAs oder dergleichen, realisiert sein. Ebenso können die benötigten Schnittstellen, beispielsweise wenn es nur um eine Übernahme von Daten aus anderen Softwarekomponenten geht, als Softwareschnittstellen ausgebildet sein. Sie können aber auch als hardwaremäßig aufgebaute Schnittstellen ausgebildet sein, die durch geeignete Software angesteuert werden.

Eine weitgehend softwaremäßige Realisierung hat den Vorteil, dass auch schon bisher für medizinische Aufgaben genutzte verwendete Rechnersysteme auf einfache Weise durch ein Software-Update nachgerüstet werden können, um auf die erfindungsgemäße Weise als Rekonstruktionseinrichtung zu arbeiten. Insofern wird die Aufgabe auch durch ein entsprechendes Computerprogrammprodukt mit einem Computerprogramm gelöst, welches direkt in eine Speichereinrichtung eines solchen Rechnersystem ladbar ist, mit Programmabschnitten, um alle Schritte des erfindungsgemäßen Verfahrens auszuführen, wenn das Computerprogramm in dem Rechnersystem ausgeführt wird.

Ein solches Computerprogrammprodukt kann neben dem Computerprogramm gegebenenfalls zusätzliche Bestandteile wie z.B. eine Dokumentation und/oder zusätzliche Komponenten auch Hardware-Komponenten, wie z.B. Hardware-Schlüssel (Dongles etc.) zur Nutzung der Software, umfassen
Zum Transport zur Speichereinrichtung des Rechnersystems und/oder zur Speicherung an dem Rechnersystem kann ein computerlesbares Medium, beispielsweise ein Memorystick, eine Festplatte oder ein sonstiger transportabler oder fest eingebauter Datenträger dienen, auf welchem die von einer Rechnereinheit einlesbaren und ausführbaren Programmabschnitte des Computerprogramms gespeichert sind. Die Rechnereinheit kann z.B. hierzu einen oder mehrere zusammenarbeitende Mikroprozessoren oder dergleichen aufweisen.

Die abhängigen Ansprüche sowie die nachfolgende Beschreibung enthalten jeweils besonders vorteilhafte Ausgestaltungen und Weiterbildungen der Erfindung. Dabei können insbesondere die Ansprüche einer Anspruchskategorie auch analog zu den abhängigen Ansprüchen einer anderen Anspruchskategorie und deren Beschreibungsteilen weitergebildet sein. Zudem können im Rahmen der Erfindung auch die verschiedenen Merkmale unterschiedlicher Ausführungsbeispiele und Ansprüche auch zu neuen Ausführungsbeispielen kombiniert werden.

In einer Ausgestaltung des erfindungsgemäßen adaptiven Verfahrens zum Erzeugen von CT-Bilddaten werden zur Erzeugung der artefaktreduzierten Bilddaten die unkorrigierten Bilddaten nur aus artefaktfreien Teilbereichen verwendet. Vorteilhaft werden die unkorrigierten Bilddaten für Teilbereiche des Gesamtbilds genutzt, in denen keine Artefakte auftreten. Mithin wird eine optimale Bildqualität für diese Bereiche erreicht, ohne zusätzlich Daten für diese Bildbereiche in einem Artefaktkorrekturverfahren zu erzeugen. Die Artefaktkorrektur beschränkt sich dann auf die artefaktbehafteten Bereiche. Auf diese Weise wird auch in den artefaktbehafteten Bereichen eine optimale Bildqualität erzielt.

Da die unkorrigierten Bilddaten in den artefaktbehafteten Bildbereichen die Bildqualität beeinträchtigen können, ist es vorteilhaft, die unkorrigierten Bilddaten für diese Bildbereiche nicht heranzuziehen, wodurch die Bildqualität des Gesamtbilds verbessert wird.

In einer bevorzugten Ausgestaltung des erfindungsgemäßen adaptiven Verfahrens zum Erzeugen von CT-Bilddaten wird bei dem Ermitteln von artefaktbehafteten Teilbereichen ermittelt, ob sich in dem Untersuchungsbereich Metallobjekte befinden, und es wird ermittelt, in welchen Teilbereichen des Untersuchungsbereichs sich gegebenenfalls die Metallobjekte befinden. Metallobjekte können insbesondere anhand der Materialdichte in dem jeweiligen Abbildungsbereich lokalisiert werden. Vorteilhaft können anhand der Lokalisierung der Metallobjekte metallartefaktbehaftete Bereiche bzw. Teilbereiche ermittelt werden, die vorzugsweise an der Position dieser Metallobjekte und darum herum auftreten.

In einer besonders bevorzugten Ausgestaltung des erfindungsgemäßen adaptiven Verfahrens zum Erzeugen von CT-Bilddaten werden vorab anhand von Echtzeitbilddaten und/oder Topogrammbilddaten potentielle Teilbereiche, welche artefaktbehaftet sein könnten, ermittelt. Werden anhand der Echtzeitbilddaten und/oder Topogrammbilddaten möglicherweise artefaktbehaftete Teilbereiche lokalisiert, so erfolgt dann zusätzlich ein Ermitteln der tatsächlich artefaktbehafteten Teilbereiche auf Basis der unkorrigierten, auf Basis der akquirierten Projektionsmessdaten rekonstruierten Bilddaten, welche den ermittelten potentiellen Teilbereichen zugeordnet sind. Vorteilhaft können anhand der Echtzeitbilddaten und/oder Topogrammbilddaten die Bereiche der rekonstruierten Bilddaten, in denen Artefakte gesucht werden, eingegrenzt werden.

Weiterhin kann auf Basis der Echtzeitbilddaten und/oder Topogrammbilddaten entschieden werden, ob überhaupt Artefakte vorliegen und ob die unkorrigierten Bilddaten nach Artefakten durchsucht werden müssen und für eine Erzeugung von artefaktkorrigierten Bilddaten zwischengespeichert werden müssen oder aufgrund von fehlenden Artefakten direkt an einen Bildspeicher bzw. eine Anzeigeeinheit weitergegeben werden können. Unter Echtzeitbilddaten sollen die auch als Real-Time-Display-Daten (abgekürzt RTD-Daten) bezeichneten Rohbilddaten verstanden werden. Durch diese Vorgehensweise kann der Aufwand zur Zwischenspeicherung von Bilddaten reduziert werden und die Prozessgeschwindigkeit erhöht werden.

In einer besonders sinnvoll anwendbaren Variante des erfindungsgemäßen adaptiven Verfahrens zum Erzeugen von CT-Bilddaten werden mithin die Echtzeitbilddaten für eine Entscheidung genutzt, ob eine artefaktbehaftete Rekonstruktion vorgenommen werden soll oder nicht.

In einer Variante des erfindungsgemäßen adaptiven Verfahrens zum Erzeugen von CT-Bilddaten wird ein Topogramm von dem Untersuchungsbereich aufgenommen und anhand des Topogramms werden potentielle Teilbereiche, welche artefaktbehaftet sein könnten, vorab ermittelt. Als Topogramm soll in diesem Zusammenhang eine niedrig aufgelöste, vor der eigentlichen Bildaufnahme durchgeführte Übersichtsaufnahme, üblicherweise eine Röntgenbildaufnahme, von dem Untersuchungsbereich verstanden werden. Sind zum Beispiel keine Echtzeitbilddaten vorhanden, so kann die Entscheidung, ob überhaupt eine Artefaktkorrektur stattfinden soll, auch anhand des Topogramms getroffen werden. Auf diese Weise kann gegebenenfalls das gesamte Bildaufnahmeverfahren beschleunigt werden.

In einer Ausgestaltung des erfindungsgemäßen adaptiven Verfahrens zum Erzeugen von CT-Bilddaten werden die unkorrigierten Bilddaten nach deren Rekonstruktion für die Erzeugung von artefaktreduzierten Bilddaten nur für den Fall, beispielsweise in einem Cache-Speicher, zwischengespeichert, dass vorab ermittelt wurde, dass überhaupt eine Artefaktreduktion durchgeführt werden muss. Auf diese Weise kann die Datenverarbeitung und Zwischenspeicherung von Bilddaten auf das notwendige Maß beschränkt werden, so dass der Prozess beschleunigt wird.

Vorzugsweise wird bei der internen Datenübermittlung zwischen der zur Rekonstruktion der artefaktreduzierten Bilddaten verwendeten Bilddatenrekonstruktionseinrichtung und einer zur Bilddarstellung verwendeten Bildanzeigeeinrichtung pro Bilddarstellung nur ein Datensatz, welcher die artefaktreduzierten Bilddaten umfasst, übertragen. D.h., es werden, um den Datenverkehr auf das nötigste zu beschränken, nur die korrigierten Bilddaten zur Bildanzeige weitergegeben. Vorteilhaft wird auf diese Weise die Geschwindigkeit des Bildgebungsverfahrens erhöht.

Die Erfindung wird im Folgenden unter Hinweis auf die beigefügten Figuren anhand von Ausführungsbeispielen noch einmal näher erläutert. Es zeigen:
FIG 1 ein Flussdiagramm, welches ein adaptives Verfahren zum Erzeugen von CT-Bilddaten gemäß einem Ausführungsbeispiel der Erfindung veranschaulicht,
FIG 2 ein Flussdiagramm, welches ein adaptives Verfahren zum Erzeugen von CT-Bilddaten gemäß einem zweiten Ausführungsbeispiel der Erfindung veranschaulicht,
FIG 3 ein Flussdiagramm, welches ein adaptives Verfahren zum Erzeugen von CT-Bilddaten gemäß einem dritten Ausführungsbeispiel der Erfindung veranschaulicht,
FIG 4 ein Blockdiagramm, mit dem eine Bilddatenrekonstruktionseinrichtung gemäß einem Ausführungsbeispiel der Erfindung dargestellt wird,
FIG 5 eine schematische Darstellung eines Computertomographiesystems gemäß einem Ausführungsbeispiel der Erfindung.

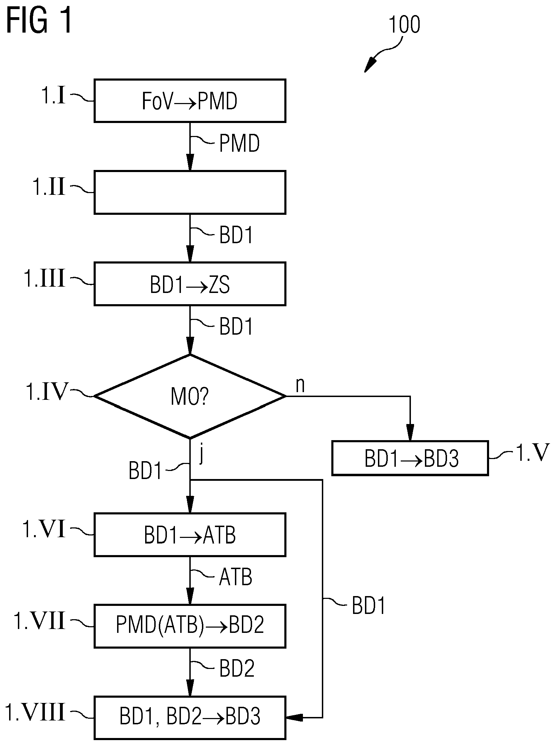

In FIG 1 ist ein Flussdiagramm 100 gezeigt, mit dem ein adaptives Verfahren zum Erzeugen von artefaktreduzierten CT-Bilddaten gemäß einem Ausführungsbeispiel der Erfindung veranschaulicht wird. Bei dem Schritt 1.I erfolgt zunächst eine Akquisition von Projektionsmessdaten PMD von einem Untersuchungsbereich FoV eines Patienten mit Hilfe einer Scaneinheit eines Computertomographiesystems. Bei dem Schritt 1.II werden weiterhin unkorrigierte, noch nicht artefaktreduzierte Bilddaten BD1 auf Basis der Projektionsmessdaten PMD rekonstruiert. Als Rekonstruktionsverfahren kann zum Beispiel ein auf der gefilterten Rückprojektion basierendes Rekonstruktionsverfahren verwendet werden. Bei dem Schritt 1.III werden die unkorrigierten rekonstruierten Bilddaten BD1 zwischengespeichert und für eine spätere Erzeugung von artefaktreduzierten Bilddaten BD3 vorgehalten. Die Zwischenspeicherung kann in einer als Cache ausgebildeten Speichereinheit ZS des Computertomographiesystems erfolgen.

Bei dem Schritt 1.IV wird dann eine Auswertung der zwischengespeicherten, unkorrigierten Bilddaten BD1 dahingehend unternommen, ob in dem Untersuchungsbereich FoV Metallobjekte MO zu finden sind. Für den Fall, dass bei dem Schritt 1.IV in dem Untersuchungsbereich FoV keine Metallobjekte MO gefunden werden konnten, was in FIG 1 mit "n" gekennzeichnet ist, wird zu dem Schritt 1.V übergegangen. Bei dem Schritt 1.V werden die rekonstruierten unkorrigierten Bilddaten BD1 als endgültige Bilddaten BD3 festgelegt und zur Weiterverarbeitung oder Begutachtung an eine Anzeigeeinheit ausgegeben werden. Für den Fall, dass bei dem Schritt 1.IV Metallobjekte MO aufgefunden wurden, was in FIG 1 mit "j" gekennzeichnet ist, wird zu dem Schritt 1.VI übergegangen.

Bei dem Schritt 1.VI werden auf Basis der detektierten Metallobjekte MO und deren Position Bereiche ATB festgelegt, in denen später eine artefaktreduzierende Rekonstruktion angewendet werden soll. Dieser Schritt kann zum Beispiel in Abhängigkeit von einer ermittelten Dichte, d.h. Materialdichte, an einer jeweiligen Position im Untersuchungsbereich FoV durchgeführt werden. Sehr dichte Bereiche weisen auf das Auftreten von Metallobjekten MO hin. Dieser Ermittlungsschritt kann vorzugsweise automatisiert anhand der ermittelten Bilddaten BD1 erfolgen. Anschließend wird bei dem Schritt 1.VII eine artefaktreduzierte Bildrekonstruktion in den metallartefaktbehafteten Teilbereichen ATB des Untersuchungsbereichs FoV durchgeführt. D.h., die artefaktreduzierende Bildrekonstruktion erfolgt auf Basis von Projektionsmessdaten PMD(ATB), welche eine Teilmenge der bei dem Schritt 1.I akquirierten Projektionsmessdaten PMD bilden und den ermittelten metallartefaktbehafteten Teilbereichen ATB des Untersuchungsbereichs FoV zugeordnet sind. Es werden also artfaktreduzierte Teilbilddaten BD2 nur von den metallartefaktbehafteten Teilbereichen ATB erzeugt. Schließlich werden bei dem Schritt 1.VIII artefaktreduzierte Bilddaten BD3 von dem gesamten Untersuchungsbereich FoV durch Kombination der unkorrigierten Bilddaten BD1 und der artfaktreduzierten Teilbilddaten BD2 erzeugt. D.h., es wird ein Bilddatensatz erzeugt, bei dem für die nicht metallartefaktbehafteten Bereiche des Untersuchungsbereichs herkömmliche Bilddaten BD1 verwendet werden und nur in den metallartefaktbehafteten Bereichen ATB artfaktreduzierte Teilbilddaten BD2 verwendet werden.

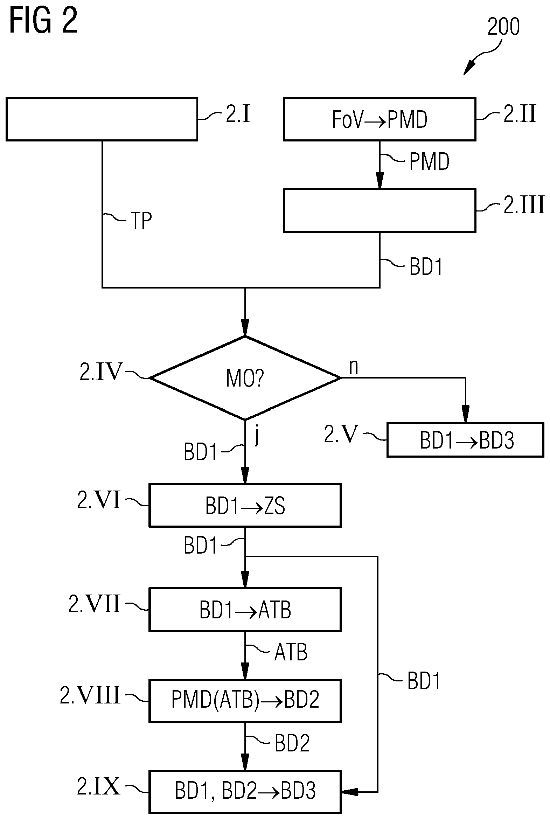

In FIG 2 ist ein Flussdiagramm 200 gezeigt, welches ein adaptives Verfahren zum Erzeugen von artefaktreduzierten CT-Bilddaten BD3 gemäß einem zweiten Ausführungsbeispiel der Erfindung veranschaulicht.

Bei dem Schritt 2.I wird zunächst mit Hilfe eines Topogramms TP eine gering aufgelöste Röntgenabbildung von einem Untersuchungsbereich FoV und auch dazu benachbarten Bereichen eines Patienten vorgenommen. Bei dem Schritt 2.II erfolgt dann eine Akquisition von Projektionsmessdaten PMD von dem Untersuchungsbereich FoV mit Hilfe einer Scaneinheit eines Computertomographiesystems. Bei dem Schritt 2.III werden weiterhin erste, noch nicht artefaktreduzierte Bilddaten BD1 auf Basis der Projektionsmessdaten PMD rekonstruiert.

Bei dem Schritt 2.IV wird dann eine Auswertung des Topogramms TP dahingehend unternommen, ob in dem Untersuchungsbereich FoV oder eventuell auch in dazu benachbarten Bereichen Metallobjekte MO zu finden sind. Für den Fall, dass bei dem Schritt 2.IV in dem Untersuchungsbereich FoV oder eventuell auch in dazu benachbarten Bereichen keine Metallobjekte MO gefunden werden konnten, was in FIG 2 mit "n" gekennzeichnet ist, wird zu dem Schritt 2.V übergegangen. Bei dem Schritt 2.V werden die rekonstruierten unkorrigierten Bilddaten BD1 als endgültige Bilddaten BD3 festgelegt und zur Weiterverarbeitung oder Begutachtung an eine Anzeigeeinheit ausgegeben. Für den Fall, dass bei dem Schritt 2.IV Metallobjekte MO aufgefunden wurden, was in FIG 2 mit "j" gekennzeichnet ist, wird zu dem Schritt 2.VI übergegangen, bei dem die rekonstruierten unkorrigierten Bilddaten BD1 zwischengespeichert und für eine spätere Erzeugung von artefaktreduzierten Bilddaten BD3 vorgehalten werden.

Die übrigen Schritte 2.VII bis 2.IX entsprechen dann den in FIG 1 veranschaulichten Schritten 1.VI bis 1.VIII und werden an dieser Stelle nicht nochmals im Einzelnen dargelegt.

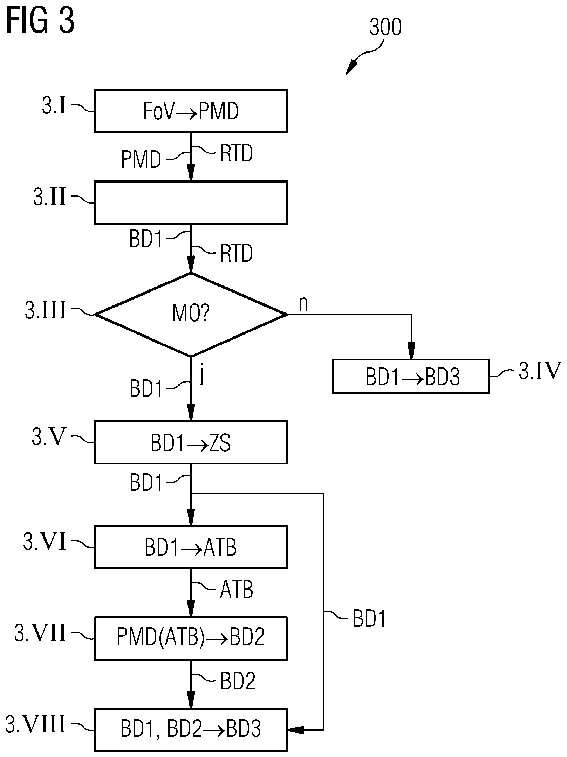

In FIG 3 ist ein Flussdiagramm 300 gezeigt, mit dem ein adaptives Verfahren zum Erzeugen von artefaktreduzierten CT-Bilddaten gemäß einem Ausführungsbeispiel der Erfindung veranschaulicht wird. Bei dem Schritt 3.I erfolgt zunächst eine Akquisition von Projektionsmessdaten PMD mit Hilfe einer Scaneinheit eines Computertomographiesystems. Zusätzlich werden jedoch bei diesem Ausführungsbeispiel auf Basis der jeweils bereits aktuell akquirierten Projektionsmessdaten PMD sogenannte Echtzeitbilddaten RTD erzeugt, welche niedrig aufgelöste Bilddaten darstellen, die während der Akquisition der Projektionsmessdaten PMD rekonstruiert werden. Bei dem Schritt 3.II werden weiterhin erste, noch nicht artefaktreduzierte Bilddaten BD1 auf Basis der Projektionsmessdaten PMD rekonstruiert.

Im weiteren Verlauf des Verfahrens wird bei dem Schritt 3.III eine Auswertung der Echtzeitbilddaten RTD dahingehend unternommen, ob in dem Untersuchungsbereich FoV Metallobjekte MO zu finden sind. Für den Fall, dass bei dem Schritt 3.III in dem Untersuchungsbereich FoV keine Metallobjekte MO gefunden werden konnten, was in FIG 1 mit "n" gekennzeichnet ist, wird zu dem Schritt 3.IV übergegangen. Bei dem Schritt 3.IV werden die rekonstruierten ersten Bilddaten BD1 als endgültige Bilddaten BD3 festgelegt und zur Weiterverarbeitung oder Begutachtung an eine Anzeigeeinheit ausgegeben werden. Für den Fall, dass bei dem Schritt 3.III Metallobjekte MO aufgefunden wurden, was in FIG 1 mit "j" gekennzeichnet ist, wird zu dem Schritt 3.V übergegangen, bei dem die unkorrigierten rekonstruierten Bilddaten BD1 zwischengespeichert und für eine spätere Erzeugung von artefaktreduzierten Bilddaten BD3 vorgehalten werden. Die Zwischenspeicherung kann in einer als Cache ausgebildeten Speichereinheit bzw. einem entsprechenden Speicherbereich einer Speichereinheit erfolgen.

Die übrigen Schritte 3.VI bis 3.VIII entsprechen dann den in FIG 1 veranschaulichten Schritten 1.VI bis 1.VIII und werden an dieser Stelle nicht nochmals im Einzelnen dargelegt.

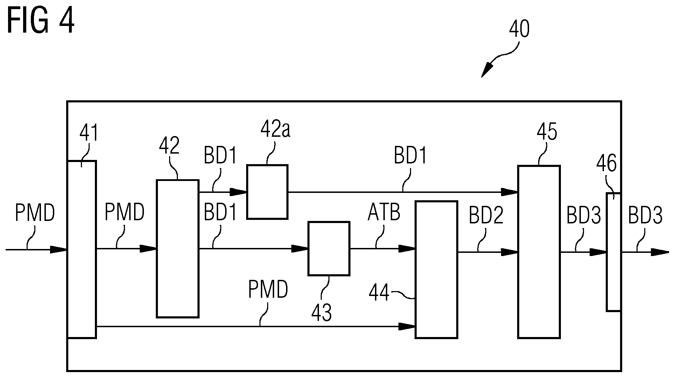

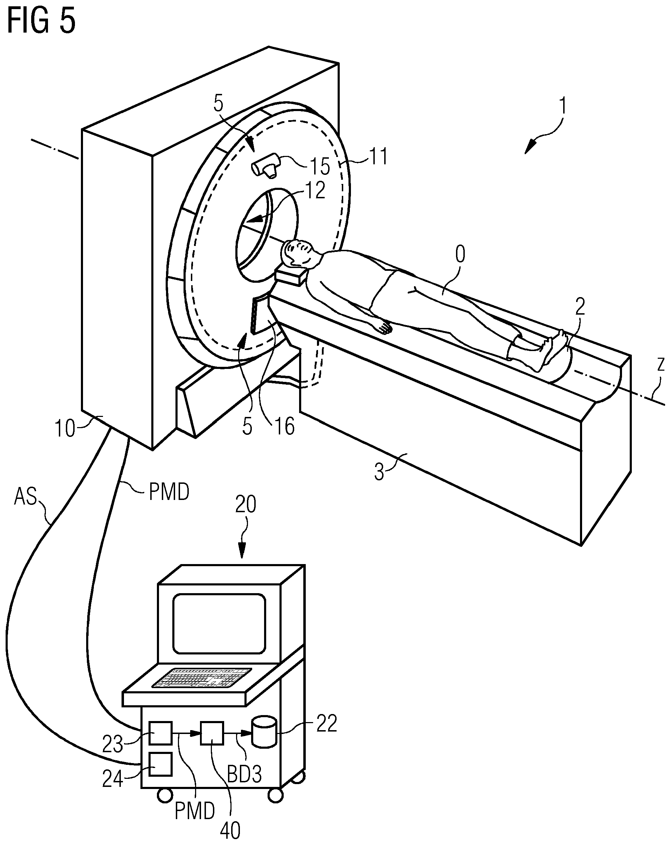

In FIG 4 ist eine Bilddatenrekonstruktionseinrichtung 40 gemäß einem Ausführungsbeispiel der Erfindung dargestellt. Die Bilddatenrekonstruktionseinrichtung 40 weist eine Eingangsschnittstelle 41 auf, welche Projektionsmessdaten PMD von einem Untersuchungsbereich FoV eines Untersuchungsobjekts O von einer Scaneinheit eines CT-Systems (siehe FIG 5) empfängt. Die Projektionsmessdaten PMD werden an eine Rekonstruktionseinheit 42 weitergeleitet, welche dazu eingerichtet ist, erste unkorrigierte Bilddaten BD1 auf Basis der Projektionsmessdaten PMD zu rekonstruieren. Die rekonstruierten Bilddaten BD1 werden dann in einer Zwischenspeichereinheit 42a, auch Cache genannt, zwischengespeichert

Teil der Bilddatenrekonstruktionseinrichtung 40 ist auch eine Teilbereichsermittlungseinheit 43, welche dazu eingerichtet ist, metallartefaktbehaftete Teilbereiche ATB des Untersuchungsbereichs FoV auf Basis der unkorrigierten Bilddaten BD1 zu ermitteln. Die Informationen bezüglich der metallartefaktbehafteten Teilbereiche ATB sowie die Projektionsmessdaten PMD werden an eine Korrektureinheit 44 übermittelt, welche dazu eingerichtet ist, eine artefaktreduzierte Bildrekonstruktion, beispielsweise mit Hilfe des bereits genannten iMAR-Artefaktkorrekturverfahrens, in den metallartefaktbehafteten Teilbereichen ATB des Untersuchungsbereichs FoV durchzuführen. Dabei werden die den metallartefaktbehafteten Teilbereichen ATB zugeordneten Projektionsmessdaten PMD für eine artefaktreduzierte Rekonstruktion von artefaktreduzierten Teilbilddaten BD2 genutzt, wobei artfaktreduzierte Teilbilddaten BD2 nur von den metallartefaktbehafteten Teilbereichen ATB erzeugt werden. Die artfaktreduzierten Teilbilddaten BD2 sowie komplementäre, zwischengespeicherte unkorrigierte Bilddaten BD1 werden an eine Bilderzeugungseinheit 45 übermittelt, welche artefaktreduzierte Bilddaten BD3 von dem gesamten Untersuchungsbereich FoV erzeugt. Dabei werden die artfaktreduzierten Teilbilddaten BD2 und die komplementären unkorrigierten Bilddaten BD1, d.h. die Bilddaten BD1, welche in den artefaktfreien Teilbereichen des Untersuchungsbereichs aufgenommen wurden, kombiniert. Die erzeugten artefaktreduzierten Bilddaten BD3 werden dann über eine Ausgabeschnittstelle 46 an eine Datenspeichereinheit oder eine Bildanzeigeeinheit (siehe FIG 5) ausgegeben.

In FIG 5 ist eine schematische Darstellung eines Computertomographiesystems 1 gemäß einem Ausführungsbeispiel der Erfindung veranschaulicht. Das Computertomographiesystem 1 umfasst die in FIG 4 veranschaulichte Bilddatenrekonstruktionseinrichtung 40, welche Teil einer Steuereinrichtung 20 des Computertomographiesystems ist. Das CT-System 1 besteht ansonsten im Wesentlichen aus einer üblichen Scaneinheit 10, in welcher an einer Gantry 11 eine Projektionsdatenakquisitionseinheit 5 mit einem Detektor 16 und einer dem Detektor 16 gegenüberliegenden Röntgenquelle 15 um einen Messraum 12 umläuft. Vor der Scaneinheit 10 befindet sich eine Patientenlagerungseinrichtung 3 bzw. ein Patiententisch 3, dessen oberer Teil 2 mit einem darauf befindlichen Patienten O zur Scaneinheit 10 verschoben werden kann, um den Patienten O durch den Messraum 12 hindurch relativ zum Detektorsystem 16 zu bewegen. Angesteuert werden die Scaneinheit 10 und der Patiententisch 3 durch die bereits erwähnte Steuereinrichtung 20, von der aus über eine übliche Steuerschnittstelle 24 Akquisitionssteuersignale AS kommen, um das gesamte System gemäß vorgegebener Messprotokolle in der herkömmlichen Weise anzusteuern.

Im Fall einer Spiralakquisition ergibt sich durch eine Bewegung des Patienten O entlang der z-Richtung, welche der Systemachse z längs durch den Messraum 12 entspricht, und den gleichzeitigen Umlauf der Röntgenquelle 15 für die Röntgenquelle 15 relativ zum Patienten O während der Messung eine Helixbahn. Parallel läuft dabei immer gegenüber der Röntgenquelle 15 der Detektor 16 mit, um Projektionsmessdaten PMD zu erfassen, die dann zur Rekonstruktion von Volumen- und/oder Schicht-Bilddaten genutzt werden. Ebenso kann auch ein sequentielles Messverfahren, durchgeführt werden, bei dem eine feste Position in z-Richtung angefahren wird und dann während eines Umlaufs, eines Teilumlaufs oder mehrerer Umläufe an der betreffenden z-Position die erforderlichen Projektionsmessdaten PMD erfasst werden, um ein Schnittbild an dieser z-Position zu rekonstruieren oder um aus den Projektionsdaten mehrerer z-Positionen Bilddaten zu rekonstruieren. Das erfindungsgemäße Verfahren ist grundsätzlich auch an anderen CT-Systemen, z.B. mit mehreren Röntgenquellen und/oder Detektoren und/oder mit einem einen vollständigen Ring bildenden Detektor, einsetzbar. Beispielsweise lässt sich das erfindungsgemäße Verfahren auch auf ein System mit unbewegtem Patiententisch und in z-Richtung bewegter Gantry (einer sogenannten Sliding Gantry) anwenden.

Die vom Detektor 16 akquirierten Projektionsmessdaten PMD (im Folgenden auch Rohdaten genannt) werden über eine Rohdatenschnittstelle 23 an die Steuereinrichtung 20 übergeben. Diese Rohdaten PMD werden dann, gegebenenfalls nach einer geeigneten Vorverarbeitung (z. B. Filterung und/oder Strahlaufhärtungskorrektur), in einer erfindungsgemäßen Bilddatenrekonstruktionseinrichtung 40 weiterverarbeitet, die in diesem Ausführungsbeispiel in der Steuereinrichtung 20 in Form von Software auf einem Prozessor realisiert ist. Diese Bilddatenrekonstruktionseinrichtung 40 rekonstruiert auf Basis der Rohdaten PMD artefaktreduzierte Bilddaten BD3 mit Hilfe des in FIG 1 bis FIG 3 veranschaulichten erfindungsgemäßen Verfahrens.

Die von der Rekonstruktionseinrichtung 40 ermittelten Bilddaten BD3 werden dann in einem Speicher 22 der Steuereinrichtung 20 hinterlegt und/oder in üblicher Weise auf dem Bildschirm der Steuereinrichtung 20 ausgegeben. Sie können auch über eine in FIG 5 nicht dargestellte Schnittstelle in ein an das Computertomographiesystem 1 angeschlossenes Netz, beispielsweise ein radiologisches Informationssystem (RIS), eingespeist und in einem dort zugänglichen Massenspeicher hinterlegt oder auf dort angeschlossenen Druckern oder Filming-Stationen als Bilder ausgegeben werden. Die Daten können so in beliebiger Weise weiterverarbeitet und dann gespeichert oder ausgegeben werden.

Die Komponenten der Bildrekonstruktionseinrichtung 40 können überwiegend oder vollständig in Form von Softwareelementen auf einem geeigneten Prozessor realisiert sein. Insbesondere können auch die Schnittstellen zwischen diesen Komponenten rein softwaremäßig ausgebildet sein. Erforderlich ist lediglich, dass Zugriffsmöglichkeiten auf geeignete Speicherbereiche bestehen, in denen die Daten geeignet zwischengelagert und jederzeit wieder aufgerufen und aktualisiert werden können.

Es wird abschließend noch einmal darauf hingewiesen, dass es sich bei den vorbeschriebenen Verfahren und Vorrichtungen lediglich um bevorzugte Ausführungsbeispiele der Erfindung handelt und dass die Erfindung vom Fachmann variiert werden kann, ohne den Bereich der Erfindung zu verlassen, soweit er durch die Ansprüche vorgegeben ist. So können die beschriebenen Verfahren und Vorrichtungen nicht nur für die medizinische Bildgebung genutzt werden, sondern sie können auch für andere, nichtmedizinische Zwecke verwendet werden. Es wird der Vollständigkeit halber auch darauf hingewiesen, dass die Verwendung der unbestimmten Artikel "ein" bzw. "eine" nicht ausschließt, dass die betreffenden Merkmale auch mehrfach vorhanden sein können. Ebenso schließt der Begriff "Einheit" nicht aus, dass diese aus mehreren Komponenten besteht, die gegebenenfalls auch räumlich verteilt sein können.

## Patentansprüche

1. Adaptives Verfahren zum Erzeugen von CT-Bilddaten, aufweisend die Schritte:
- Akquirieren von Projektionsmessdaten (PMD) von einem Untersuchungsbereich (FoV) eines Untersuchungsobjekts (O),
- Erzeugen von unkorrigierten Bilddaten (BD1, TP, RTD) von dem Untersuchungsbereich (FoV),
- Ermitteln von artefaktbehafteten Teilbereichen (ATB) des Untersuchungsbereichs (FoV) auf Basis zumindest eines Teils der unkorrigierten Bilddaten (BD1, TP, RTD),
- Durchführen einer artefaktreduzierten Bildrekonstruktion in den artefaktbehafteten Teilbereichen (ATB) des Untersuchungsbereichs (FoV), wobei artfaktreduzierte Teilbilddaten (BD2) nur von den artefaktbehafteten Teilbereichen (ATB) erzeugt werden,
- Erzeugen von artefaktreduzierten Bilddaten (BD3) von dem gesamten Untersuchungsbereich (FoV) durch Kombination zumindest eines Teils der unkorrigierten Bilddaten (BD1) und der artefaktreduzierten Teilbilddaten (BD2).

2. Verfahren nach Anspruch 1, wobei bei dem Schritt des Erzeugens von unkorrigierten Bilddaten (BD1, TP, RTD) unkorrigierte Bilddaten (BD1) auf Basis der akquirierten Projektionsmessdaten (PMD) rekonstruiert werden und zumindest ein Teil dieser rekonstruierten unkorrigierten Bilddaten (BD1) bei dem Schritt des Erzeugens von artefaktreduzierten Bilddaten (BD3) mit den artefaktreduzierten Teilbilddaten (BD2) kombiniert werden.

3. Verfahren nach einem der Ansprüche 1 oder 2, wobei die unkorrigierten Bilddaten (BD1, TP, RTD) Topogrammbilddaten (TP) und/oder Echtzeitbilddaten (RTD) umfassen.

4. Verfahren nach einem der vorstehenden Ansprüche, wobei zur Erzeugung der artefaktreduzierten Bilddaten (BD3) die unkorrigierten Bilddaten (BD1) nur aus artefaktfreien Teilbereichen verwendet werden.

5. Verfahren nach einem der vorstehenden Ansprüche, wobei das Ermitteln von artefaktbehafteten Teilbereichen (ATB) die Schritte umfasst:
- Ermitteln, ob sich in dem Untersuchungsbereich (FoV) Metallobjekte befinden, und
- Ermitteln, in welchen Teilbereichen des Untersuchungsbereichs (FoV) sich gegebenenfalls die Metallobjekte (MO) befinden.

6. Verfahren nach einem der Ansprüche 3 bis 5, wobei die artefaktbehafteteten Teilbereiche (ATB) auf Basis der Echtzeitbilddaten (RTD) und/oder der Topogrammbilddaten (TP) ermittelt werden.

7. Verfahren nach einem der Ansprüche 3 bis 5, wobei vorab anhand der Echtzeitbilddaten (RTD) und/oder der Topogrammbilddaten (TP) potentielle Teilbereiche, welche artefaktbehaftet sein könnten, ermittelt werden und das Ermitteln von artefaktbehafteten Teilbereichen (ATB) auf Basis von rekonstruierten unkorrigierten Bilddaten (BD1) erfolgt, welche den ermittelten potentiellen Teilbereichen zugeordnet sind.

8. Verfahren nach einem der Ansprüche 3 bis 5, wobei die Echtzeitbilddaten (RTD) und/oder Topogrammbilddaten (TP) für eine Entscheidung genutzt werden, ob eine artefaktbehaftete Rekonstruktion vorgenommen werden soll oder nicht.

9. Verfahren nach Anspruch 8, wobei rekonstruierte unkorrigierte Bilddaten (BD1) nach deren Rekonstruktion für die Erzeugung von artefaktreduzierten Bilddaten (BD3) nur für den Fall zwischengespeichert werden, dass vorab ermittelt wurde, dass eine Artefaktreduktion durchgeführt werden muss.

10. Verfahren nach einem der vorstehenden Ansprüche, wobei bei der internen Datenübermittlung zwischen einer zur Rekonstruktion der artefaktreduzierten Bilddaten (BD3) verwendeten Bilddatenrekonstruktionseinrichtung (40) und einer Bildanzeigeeinrichtung pro Bilddarstellung nur ein Datensatz, welcher die artefaktreduzierten Bilddaten (BD3) umfasst, übertragen wird.

11. Bilddatenrekonstruktionseinrichtung (40), aufweisend:
- eine Eingangsschnittstelle (41) zum Akquirieren von Projektionsmessdaten (PMD) von einem Untersuchungsbereich (FoV) eines Untersuchungsobjekts (O),
- eine Rekonstruktionseinheit (42) zum Rekonstruieren von unkorrigierten Bilddaten (BD1) auf Basis der Projektionsmessdaten (PMD),
- eine Teilbereichsermittlungseinheit (43) zum Ermitteln von artefaktbehafteten Teilbereichen (ATB) des Untersuchungsbereichs (FoV) auf Basis von unkorrigierten Bilddaten (BD1, TP, RTD),
- eine Korrektureinheit (44) zum Durchführen einer artefaktreduzierten Bildrekonstruktion in den artefaktbehafteten Teilbereichen (ATB) des Untersuchungsbereichs (FoV), wobei artfaktreduzierte Teilbilddaten (BD2) nur von den artefaktbehafteten Teilbereichen (ATB) erzeugt werden,
- eine Bilderzeugungseinheit (45) zum Erzeugen von artefaktreduzierten Bilddaten (BD3) von dem gesamten Untersuchungsbereich (FoV) durch Kombination zumindest eines Teils der rekonstruierten unkorrigierten Bilddaten (BD1) und der artfaktreduzierten Teilbilddaten (BD2).

12. Computertomographiesystem (1), aufweisend:
- eine Scaneinheit (10) zum Erfassen eines zu untersuchenden Bereichs (FoV) eines Untersuchungsobjekts (O),
- eine Steuereinrichtung (20) zum Ansteuern der Scaneinheit (10),
- eine Bilddatenrekonstruktionseinrichtung (40) nach Anspruch 11.

13. Computerprogrammprodukt mit einem Computerprogramm, welches direkt in eine Rechnereinheit eines Computertomographiesystems ladbar ist, mit Programmabschnitten, um alle Schritte des Verfahrens nach einem der Ansprüche 1 bis 10 auszuführen, wenn das Computerprogramm in der Rechnereinheit ausgeführt wird.

14. Computerlesbares Medium, auf welchem von einer Rechnereinheit ausführbare Programmabschnitte gespeichert sind, um alle Schritte des Verfahrens nach einem der Ansprüche 1 bis 10 auszuführen, wenn die Programmabschnitte von der Rechnereinheit ausgeführt werden.

## Claims

1. Adaptive method for generating CT image data, having the steps:
- acquiring projection measurement data (PMD) of an examination region (FoV) of an examination object (O),
- generating uncorrected image data (BD1, TP, RTD) of the examination region (FoV),
- determining artifact-affected subregions (ATB) of the examination region (FoV) on the basis of at least one part of the uncorrected image data (BD1, TP, RTD),
- carrying out an artifact-reduced image reconstruction in the artifact-affected subregions (ATB) of the examination region (FoV), wherein artifact-reduced subimage data (BD2) is only generated from the artifact-affected subregions (ATB),
- generating artifact-reduced image data (BD3) of the entire examination region (FoV) by combining at least one part of the uncorrected image data (BD1) and the artifact-reduced subimage data (BD2).

2. Method according to claim 1, wherein in the step of generating uncorrected image data (BD1, TP, RTD), uncorrected image data (BD1) is reconstructed on the basis of the acquired projection measurement data (PMD) and at least one part of this reconstructed, uncorrected image data (BD1) is combined with the artifact-reduced subimage data (BD2) in the step of generating artifact-reduced image data (BD3).

3. Method according to one of claims 1 or 2, wherein the uncorrected image data (BD1, TP, RTD) comprises topogram image data (TP) and/or real-time image data (RTD).

4. Method according to one of the preceding claims, wherein in order to generate the artifact-reduced image data (BD3), the uncorrected image data (BD1) is only used from artifact-free subregions.

5. Method according to one of the preceding claims, wherein determining artifact-affected subregions (ATB) includes the steps:
- determining whether metal objects are located in the examination region (FoV), and
- determining the subregions of the examination region (FoV) in which the metal objects (MO) may possibly be located.

6. Method according to one of claims 3 to 5, wherein the artifact-affected subregions (ATB) are determined on the basis of the real-time image data (RTD) and/or the topogram image data (TP).

7. Method according to one of claims 3 to 5, wherein potential subregions, which may be artifact-affected, are determined in advance by means of the real-time image data (RTD) and/or the topogram image data (TP) and artifact-affected subregions (ATB) are determined on the basis of reconstructed, uncorrected image data (BD1), which is assigned to the potential subregions determined.

8. Method according to one of claims 3 to 5, wherein the real-time image data (RTD) and/or topogram image data (TP) is used to decide on whether or not an artifact-affected reconstruction is to be performed.

9. Method according to claim 8, wherein after its reconstruction for the generation of artifact-reduced image data (BD3), reconstructed, uncorrected image data (BD1) is buffered only in the event that it was previously determined that an artifact reduction has to be carried out.

10. Method according to one of the preceding claims, wherein with the internal data transmission between an image data reconstruction device (40) used to reconstruct the artifact-reduced image data (BD3) and an image display device per image display, only one data record, which comprises the artifact-reduced image data (BD3), is transmitted.

11. Image data reconstruction device (40), having:
- an input interface (41) for acquiring projection measurement data (PMD) of an examination region (FoV) of an examination object (O),
- a reconstruction unit (42) for reconstructing uncorrected image data (BD1) on the basis of the projection measurement data (PMD),
- a subregion determination unit (43) for determining artifact-affected subregions (ATB) of the examination region (FoV) on the basis of uncorrected image data (BD1, TP, RTD),
- a correction unit (44) for carrying out an artifact-reduced image reconstruction in the artifact-affected subregions (ATB) of the examination region (FoV), wherein only artifact-reduced subimage data (BD2) of the artifact-affected subregions (ATB) is generated,
- an image generation unit (45) for generating artifact-reduced image data (BD3) of the entire examination region (FoV) by combining at least one part of the uncorrected image data (BD1) and the artifact-reduced subimage data (BD2).

12. Computed tomography system (1), having:
- a scanner unit (10) for capturing a region (FoV), to be examined, of an examination object (O),
- a control device (20) for controlling the scanner unit (10),
- an image data reconstruction device (40) according to claim 11.

13. Computer program product having a computer program which is directly loadable into a computer unit of a computed tomography system, having program portions in order to carry out all the steps of the method according to one of claims 1 to 10 when the computer program is executed in the computer unit.

14. Computer-readable medium on which program portions are stored which are configured to be executable by a computer unit in order to carry out all the steps of the method according to one of claims 1 to 10 when the program portions are executed by the computer unit.

## Revendications

1. Procédé adaptatif de production de données d'image CT, comportant les stades :
- acquisition de données (PMD) de mesure de projection d'une région (FoV) à examiner d'un objet (O) à examiner,
- production de données (BD1, TP, RTD) d'image non corrigées de la région (FoV) à examiner,
- détermination de régions (ATB) partielles entachées d'artefacts de la région (FoV) à examiner, sur la base d'au moins une partie des données (BD1, TP, RTD) d'image non corrigées,
- mise en œuvre d'une reconstruction d'image réduite en artefacts dans les régions (ATB) partielles entachées d'artefacts de la région (FoV) à examiner, des données (BD2) d'image partielles réduites en artefacts étant produites seulement des régions (ATB) partielles entachées d'artefacts,
- production de données (BD3) d'image réduites en artefacts de toute la région (FoV) à examiner par combinaison d'au moins une partie des données (BD1) d'image non corrigées et des données (BD2) d'image partielles réduites en artefacts.

2. Procédé suivant la revendication 1, dans lequel, dans le stade de la production de données (BD1, TP, RTD) d'images non corrigées, on reconstruit des données (BD1) d'images non corrigées sur la base des données (PMD) de projection acquises et on combine au moins une partie de ces données (BD1) d'image non corrigées reconstruites dans le stade de la production de données (BD3) d'image réduites en artefacts, aux données (BD2) d'image partielles réduites en artefacts.

3. Procédé suivant l'une des revendications 1 ou 2, dans lequel les données (BD1, TP, RTD) d'image non corrigées comprennent des données (TP) d'image de topogramme et/ou des données (RTD) d'image en temps réel.

4. Procédé suivant l'une des revendications précédentes, dans lequel, pour produire les données (BD3) d'image réduites en artefacts, on utilise les données (BD1) d'image non corrigées seulement des régions partielles sans artefact.

5. Procédé suivant l'une des revendications précédentes, dans lequel la détermination des régions (ATB) partielles entachées d'artefacts comprend les stades :
- détermination du point de savoir si des objets métalliques se trouvent dans la région (FoV) à examiner, et
- détermination des régions partielles de la région (FoV) à examiner, dans lesquelles se trouvent, éventuellement, les objets (MO) métalliques.

6. Procédé suivant l'une des revendications 3 à 5, dans lequel on détermine les régions (ATB) partielles entachées d'artefacts sur la base des données (RTD) d'image en temps réel et/ou des données (TP) d'image de topogramme.

7. Procédé suivant l'une des revendications 3 à 5, dans lequel on détermine auparavant, à l'aide des données (RTD) d'image en temps réel et/ou des données (TP) d'image de topogramme, des régions partielles potentielles, qui pourraient être entachées d'artefacts, et la détermination des régions (ATB) partielles entachées d'artefacts s'effectue sur la base de données (BD1) d'image non corrigées reconstruites, qui sont affectées aux régions partielles potentielles déterminées.

8. Procédé suivant l'une des revendications 3 à 5, dans lequel on utilise les données (RTD) d'image en temps réel et/ou les données (TP) d'image de topogramme pour décider du point de savoir s'il faut ou non effectuer une reconstruction entachée d'artefacts.

9. Procédé suivant la revendication 8, dans lequel on ne met des données (BD1) d'image non corrigées reconstruites, après leur reconstruction, pour la production de données (BD3) d'image réduites en artefacts, en mémoire tampon, que dans le cas où il a été déterminé auparavant qu'il faut effectuer une réduction d'artefacts.

10. Procédé suivant l'une des revendications précédentes, dans lequel, lors de la transmission interne de données entre un dispositif (40) de reconstruction de données d'image, utilisé pour la reconstruction des données (BD3) d'image réduites en artefacts, et un dispositif d'affichage d'image, on ne transmet, par représentation d'image, qu'un jeu de données, qui comprend les données (BD3) d'image réduites en artefacts.

11. Dispositif (40) de reconstruction de données d'image, comportant :
- une interface (41) d'entrée pour l'acquisition de données (PMD) de projection d'une région (FoV) à examiner d'un objet (O) à examiner,
- une unité (42) de reconstruction pour le reconstruction de données (BD1) d'image non corrigées sur la base des données (PMD) de mesure de projection,
- une unité (43) de détermination de régions partielles pour la détermination de régions (ATB) partielles entachées d'artefacts de la région (FoV) à examiner sur la base de données (BD1, TP, RTD) d'image non corrigées,
- une unité (44) de correction pour effectuer une construction d'image réduite en artefacts dans les régions (ATB) partielles entachées d'artefacts de la région (FoV) à examiner, des données (BD2) d'image partielles réduites en artefacts étant produites seulement des régions (ATB) partielles entachées d'artefacts,
- une unité (45) de production d'image pour la production de données (BD3) d'image réduites en artefacts de toute la région (FoV) à examiner, par combinaison d'au moins une partie des données (BD1) d'image non corrigées reconstruites et des données (BD2) d'image partielles réduites en artefacts.

12. Système (1) de tomographie assisté par ordinateur, comportant :
- une unité (10) de scan pour la détection d'une région (FoV) à examiner d'un objet (O) à examiner,
- un dispositif (20) de commande pour commander l'unité (10) de scan,
- un dispositif (40) de reconstruction de données d'image suivant la revendication 11.

13. Produit de programme d'ordinateur, comprenant un programme d'ordinateur, qui peut être chargé directement dans l'unité informatique d'un système de tomographie assisté par ordinateur et qui a des parties de programme pour exécuter tous les stades du procédé suivant l'une des revendications 1 à 10, lorsque le programme d'ordinateur est réalisé dans l'unité informatique.

14. Support déchiffrable par ordinateur, sur lequel des parties de programme, pouvant être exécutées par une unité informatique, sont mises en mémoire pour effectuer tous les stades du procédé suivant l'une des revendications 1 à 10, lorsque les parties de programme sont réalisées par l'unité informatique.
